# EUROPEAN PATENT APPLICATION

(11) **EP 4 711 448 A1**
(43) Date of publication of application: **18.03.2026**
(21) Application number: 23936010.0
(22) Date of filing: 09.05.2023
(51) Int. Cl.: C12N 5/077, A61K 35/12, A61K 38/42, A61P 3/10, A61P 13/12

(54) **METHOD FOR PROMOTING GENERATION OF MICROVESICLES CONTAINING MITOCHONDRIA AND MEDICAL USE THEREOF**

(71) Applicant: KingBios Co., Ltd., Taipei City, Taiwan (TW)
(72) Inventor: WANG, Yi-Chuang, Taipei City Taiwan (TW)
(74) Representative: Petraz, Gilberto Luigi
(86) International application number: PCT/CN2023/092873
(87) International publication number: WO 2024/229682

(57) **Abstract**

A method for promoting generation of a mitochondria-containing microvesicle and a pharmaceutical use thereof. Specifically, provided is a use of a combination of a mitochondrial generation promoting factor and an extracellular vesicle generation promoting factor, for use in promoting generation of the mitochondria-containing microvesicle. Also provided is a method for generating an extracellular vesicle composition: co-culturing a cell having mitochondria with the mitochondrial generation promoting factor and the extracellular vesicle generation promoting factor to obtain the extracellular vesicle composition rich in the mitochondria-containing microvesicle. Adding the above factors may promote the cell to generate the mitochondria-containing microvesicle. The collected mitochondria are encapsulated by the microvesicle, and therefore can be easily stored and transferred into the cell. Also provided are an isolated extracellular vesicle composition, a pharmaceutical composition comprising the same, and a use thereof in preparing a medicine for treating diabetes and/or renal injury.

## Description

### Technical Field

The present invention is related to a use of using a combination of a mitochondrial generation promoting factor and an extracellular vesicle generation promoting factor. Besides, the present invention is also related to a method for making a cell to generate an extracellular vesicle composition rich in a mitochondria-containing microvesicle, an isolated extracellular vesicle composition, a pharmaceutical composition comprising the isolated extracellular vesicle composition, and a use thereof.

### Background Technology

Stem cells are undifferentiated precursor cells that have the ability to differentiate into cells with different functions and replicate to produce more identical stem cells. Because stem cells have the ability to replicate and differentiate into other types of cells, they are considered useful for treating diseases, and people have started trying to take stem cells as a medicine. Stem cell therapy, defined as the use of stem cells to treat a variety of diseases, can be traced back to bone marrow transplantation in 1968. While the medical technique of marrow replacement involves the transplantation of "hematopoietic stem cells", it differs from today's treatment of mesenchymal stem cell transplantation but can still be considered as a beginning of allogeneic stem cell therapy. Mesenchymal stem cells were tested in numerous animal experiments to obtain promising results, and people gradually tried to treat human diseases with stem cells after 2000. For example, in 2003, "bone marrow stem cells" were implanted into the left ventricle to repair myocardial tissues. Application to human spinal cord injuries has shown a significant recovery in mobility. In stroke treatment, they can relieve acute inflammation and long-term brain degeneration, promoting long-term functional recovery. Studies on brain injuries have demonstrated their ability to alleviate symptoms and improve mobility and long-term memory. Later, adult stem cells derived from fat were also shown to be effective in treating neurological diseases. While time and evidences have proven the effectiveness of stem cell therapy, the original theory of differentiation replacement has been inconsistent with experimental research results, so the therapeutic mechanism of mesenchymal stem cells has remained uncertain.

Recent studies have confirmed that the therapeutic effects of stem cells come from substances secreted by stem cells, wherein mitochondria are transferred to damaged cells through tunnels, gap junctions, microvesicles, and cell fusions. The damaged cells then acquire these exogenous mitochondria, which is the primary reason for the stem cells' therapeutic effects. Numerous papers, such as one published by Paliwal et al. (2018), have also demonstrated that the regenerative capacity of mesenchymal stem cells comes from mitochondrial transport. In the same year, Wang et al. (2018) proposed that mitochondrial transport of stem cells is a novel therapeutic technique for tissue damage.

Current studies have also confirmed that stem cells with superior mitochondrial quality possess greater therapeutic potential. Later research has directly confirmed that whole stem cells are not required for treatment. Injecting the isolated mitochondria directly into the lungs can treat lung inflammation and restore fibrotic alveoli. Alternatively, injecting free mitochondria directly into the brain can treat stroke to reduce cerebral infarction size and multiple system atrophy (MSA) to reduce brain damage and improve behavioral abilities.

Therefore, transporting healthy mitochondria into damaged cells can promote cell regeneration, growth, and activation, improving aging or various degenerative diseases caused by mitochondrial damage. However, the greatest technical obstacle to achieving such an effective treatment is the lack of methods for mass-producing mitochondria. Furthermore, the isolated mitochondria, upon leaving the intracellular environment, quickly become damaged and die, making them difficult to be preserved. Consequently, the purified mitochondrial preparations remain difficult to manufacture, mass-produce, and preserve for extended periods.

### Invention Content

Given that mitochondria cannot be mass-produced in the prior arts, and they are difficult to be preserved even if they are produced, the innovation of the present invention lies in the simple, large-scale, and continuous mass production of mitochondrial preparations. The produced mitochondria are easier to be preserved for a long time. The structure of the vesicles and the components therein can not only protect the mitochondria from damage, but also promote the mitochondria to enter damaged cells, thereby enhancing the therapeutic ability, and solving all technical barriers to the practical application of purified mitochondrial preparations at once.

In order to achieve the aforementioned objective, the present invention provides a use of a combination of a mitochondrial generation promoting factor and an extracellular vesicle generation promoting factor, for use in promoting a cell having mitochondria to generate a mitochondria-containing microvesicle.

The use of the present invention may be used for non-therapeutic objectives.

Through using a combination of the mitochondrial generation promoting factor and the extracellular vesicle generation promoting factor, the present invention can mass-produce the mitochondria-containing microvesicle. The structure of the microvesicle can promote the mitochondria to enter damaged cells for repair. Moreover, the collected mitochondria are already encapsulated by the microvesicle and protected by small molecule ribonucleic acid (RNA), so they are easier to be preserved.

According to the present invention, the cell having mitochondria may generate an extracellular vesicle and the mitochondria-containing microvesicle.

Preferably, a ratio of an addition amount of the mitochondrial generation promoting factor and an addition amount of the extracellular vesicle generation promoting factor is 1:1 to 1:1000, such as 1:1 to 1:8, 1:1 to 1:6, 1:1 to 1:4, or 1:100, 1:300, 1:600, 1:900. More preferably, the ratio of an addition amount of the mitochondrial generation promoting factor and an addition amount of the extracellular vesicle generation promoting factor is 1:1 to 1:5.

Preferably, the mitochondrial generation promoting factor may be selected from the group consisting of heme oxygenase-1 (HO-1), cobalt protoporphyrin IX (CoPPIX), hemin, hemin derivatives (such as hemin chloride), hemoprotein, schistocyte, mitochondrial structure fragments, cell tissue fragments, and a culture environment having 5% to 20% oxygen concentration.

Preferably, the extracellular vesicle generation promoting factor may be selected from the group consisting of ethanol; EP4 antagonist, such as GW627368X; endosomal sorting complex required for transport (ESCRT); arrestin domain-containing protein 1 (ARRDC1); associated protein's tumor suppressor gene 101 (TSG101); and cytochalasin B. The extracellular vesicle generation promoting factor may promote the cell to generate microvesicles and exosomes.

Preferably, the cell having mitochondria is a mesenchymal stem cell, a hematopoietic stem cell, a bone marrow stem cell, a liver cell, or another somatic cell. More preferably, the cell having mitochondria is a mesenchymal stem cell. Preferably, the cell having mitochondria is a newborn mesenchymal stem cell or an embryonic stem cell.

In order to achieve the aforementioned objective, the present invention further provides a method for generating an extracellular vesicle composition rich in a mitochondria-containing microvesicle, comprising:
Step (1) providing a cell having mitochondria;
Step (2) co-culturing the cell having mitochondria in a culture medium added with a mitochondrial generation promoting factor and an extracellular vesicle generation promoting factor to obtain the extracellular vesicle composition rich in the mitochondria-containing microvesicle, wherein based on a total amount of the extracellular vesicle, the extracellular vesicle composition rich in the mitochondria-containing microvesicle comprises 0.2% or more of the mitochondria-containing microvesicle.
Preferably, step (2) comprises:
   Step (2-1) co-culturing the cell having mitochondria in the culture medium added with the mitochondrial generation promoting factor and the extracellular vesicle generation promoting factor; and
   Step (2-2) isolating an extracellular vesicle composition from the culture medium for co-culturing to obtain the extracellular vesicle composition rich in the mitochondria-containing microvesicle, wherein based on a total amount of the extracellular vesicle, the extracellular vesicle composition rich in the mitochondria-containing microvesicle comprises 0.2% or more of the mitochondria-containing microvesicle. That is, removing the cell having mitochondria and the culture medium to obtain the extracellular vesicle composition rich in the mitochondria-containing microvesicle. That is, 0.2% or more of the extracellular vesicle is the mitochondria-containing microvesicle.

Preferably, step (2) is co-culturing the cell having mitochondria in the culture medium added with the mitochondrial generation promoting factor first, and then added with the extracellular vesicle generation promoting factor, to obtain the extracellular vesicle composition rich in the mitochondria-containing microvesicle, wherein based on a total amount of the extracellular vesicle, the extracellular vesicle composition rich in the mitochondria-containing microvesicle comprises 0.2% or more of the mitochondria-containing microvesicle. In another embodiment, step (2) is co-culturing the cell having mitochondria in the culture medium added with the extracellular vesicle generation promoting factor first, and then added with the mitochondrial generation promoting factor, obtain the extracellular vesicle composition rich in the mitochondria-containing microvesicle, wherein based on a total amount of the extracellular vesicle, the extracellular vesicle composition rich in the mitochondria-containing microvesicle comprises 0.2% or more of the mitochondria-containing microvesicle. That is, 0.2% or more of the extracellular vesicle is the mitochondria-containing microvesicle.

Preferably, an amount of the added mitochondrial generation promoting factor makes a concentration of 0.1 nanograms (ng)/milliliter (mL) to 100,000 ng/mL of the mitochondrial generation promoting factor in the culture medium. Preferably, the amount of the added mitochondrial generation promoting factor makes a concentration of 0.1 ng/mL to 10,000 ng/mL of the mitochondrial generation promoting factor in the culture medium, such as 10 ng/mL, 50 ng/mL, 100 ng/mL, 500 ng/mL, or 1,000 ng/mL.

Preferably, the mitochondrial generation promoting factor may be selected from the group consisting of heme oxygenase-1; CoPPIX; hemin; hemin derivatives, such as hemin chloride; hemoprotein; schistocyte; mitochondrial structure fragments; cell tissue fragments; and a culture environment having 5% to 20% oxygen concentration, wherein the oxygen concentration may be 7% to 18% or 10% to 15%, or may be 12%, 13%, or 14%.

Preferably, an addition amount of heme oxygenase-1 makes a concentration of 0.1 ng/mL to 300 ng/mL of heme oxygenase-1 in the culture medium. Preferably, the addition amount of heme oxygenase-1 makes a concentration of 1 ng/mL to 100 ng/mL of heme oxygenase-1 in the culture medium, such as 10 ng/mL, 30 ng/mL, 50 ng/mL, or 70 ng/mL.

Preferably, an addition amount of CoPPIX makes a concentration of 0.2 µmol/kilogram (kg) to 100 µmol/kg of CoPPIX in the culture medium. Preferably, the addition amount of CoPPIX makes a concentration of 1 µmol/kg to 70 µmol/kg of CoPPIX in the culture medium, such as 5 µmol/kg, 10 µmol/kg, 30 µmol/kg, or 50 µmol/kg.

Preferably, an addition amount of hemin or hemin derivatives makes a concentration of 0.2 µmol/kg to 100 µmol/kg of hemin or hemin derivatives in the culture medium. Preferably, the addition amount of hemin or hemin derivatives makes a concentration of 1 µmol/kg to 80 µmol/kg of hemin or hemin derivatives in the culture medium, such as 10 µmol/kg, 20 µmol/kg, 50 µmol/kg, or 70 µmol/kg.

Preferably, an addition amount of the extracellular vesicle generation promoting factor makes a concentration of 10⁻⁷ mmol/liter (mM) to 800 mM of the extracellular vesicle generation promoting factor in the culture medium. Preferably, the addition amount of the extracellular vesicle generation promoting factor makes a concentration of 10⁻⁵ mM to 500 mM of the extracellular vesicle generation promoting factor in the culture medium, such as 10⁻⁴ mM, 10⁻² mM, 1 mM, 10 mM, 50 mM, 100 mM, or 300 mM.

Preferably, the extracellular vesicle generation promoting factor is selected from the group consisting of ethanol; EP4 antagonist, such as GW627368X; endosomal sorting complex required for transport; ARRDC 1; associated protein's tumor suppressor gene 101; and cytochalasin B. The extracellular vesicle generation promoting factor may promote the cell to generate microvesicles and exosomes.

Preferably, an addition amount of ethanol makes a concentration of 10 mM to 100 mM of ethanol in the culture medium. More preferably, the addition amount of ethanol makes a concentration of 20 mM to 70 mM of ethanol in the culture medium, such as 30 mM, 40 mM, 50 mM, or 60 mM.

Preferably, an addition amount of EP4 antagonist makes a concentration of 0.2 micrograms (µg)/mL to 500 µg/mL of EP4 antagonist in the culture medium. Preferably, the addition amount of EP4 antagonist makes a concentration of 1 µg/mL to 200 µg/mL of EP4 antagonist in the culture medium, such as 10 µg/mL, 50 µg/mL, 70 µg/mL, 100 µg/mL, or 150 µg/mL.

Preferably, an addition amount of endosomal sorting complex required for transport makes a concentration of 0.2 nmol/liter (nM) to 500 nM of endosomal sorting complex required for transport in the culture medium. Preferably, the addition amount of endosomal sorting complex required for transport makes a concentration of 1 nM to 300 nM of endosomal sorting complex required for transport in the culture medium, such as 5 nM, 10 nM, 50 nM, or 100 nM.

Preferably, an addition amount of ARRDC1 makes a concentration of 0.3 µmol/liter (µM) to 600 µM of ARRDC1 in the culture medium. Preferably, the addition amount of ARRDC1 makes a concentration of 1 µM to 300 µM of ARRDC1 in the culture medium, such as 10 µM, 50 µM, or 150 µM.

Preferably, an addition amount of associated protein's tumor suppressor gene 101 makes a concentration of 10⁻¹⁰ mol/liter (M) to 10⁻⁶ M of associated protein's tumor suppressor gene 101 in the culture medium. Preferably, the addition amount of associated protein's tumor suppressor gene 101 makes a concentration of 10⁻⁹ M to 10⁻⁷ M of associated protein's tumor suppressor gene 101 in the culture medium, such as 10⁻⁸ M.

Preferably, an addition amount of cytochalasin B makes a concentration of 10⁻⁷ M to 10⁻⁴ M of cytochalasin B in the culture medium. Preferably, the addition amount of cytochalasin B makes a concentration of 10⁻⁶ M to 10⁻⁵ M of cytochalasin B in the culture medium.

According to the present invention, in the above method for generating the extracellular vesicle composition rich in the mitochondria-containing microvesicle, the culture conditions of the cell having mitochondria may be adjusted by those skilled in the art as needed.

Preferably, the co-culturing is culturing under 5% carbon dioxide (CO₂) at 35°C to 39°C for 60 hours to 80 hours, preferably 70 hours to 75 hours.

In order to achieve the aforementioned objective, the present invention further provides an isolated extracellular vesicle composition, wherein based on the total amount of the extracellular vesicle, the isolated extracellular vesicle composition comprises 0.2% or more of the mitochondria-containing microvesicle.

The isolated extracellular vesicle composition further comprises a microvesicle and an exosome. The extracellular vesicle which may encapsulate the mitochondria is a microvesicle. Combining the mitochondria-containing microvesicle with the another microvesicle or exosome mentioned above may enhance their therapeutic effects.

Preferably, based on the total amount of the extracellular vesicle, the isolated extracellular vesicle composition comprises 0.5% or more, 1% or more, 2% or more, or 3% or more of the mitochondria-containing microvesicle. More preferably, based on the total amount of the extracellular vesicle, the isolated extracellular vesicle composition comprises 4% or more of the mitochondria-containing microvesicle, for example, 4.2% or more, 4.4% or more, 4.6% or more, or 4.8% or more of the mitochondria-containing microvesicle.

The present invention further provides a pharmaceutical composition, comprising the aforementioned isolated extracellular vesicle composition comprising the mitochondria-containing microvesicle and a pharmaceutically acceptable excipient.

The present invention further provides a use of an isolated extracellular vesicle composition, for use in preparing a medicine for treating or relieving diabetes. The medicine comprises an effective dose of the isolated extracellular vesicle composition and a pharmaceutically acceptable excipient, wherein based on the total amount of the extracellular vesicle, the isolated extracellular vesicle composition comprises 0.2% or more of the mitochondria-containing microvesicle.

Preferably, the diabetes is type 2 diabetes.

The effective dose of the present invention refers to an amount that is effective in achieving the desired treatment or alleviation of diabetes in terms of dosage and for the required time period. According to the present invention, it means that by administering a specific range of amount of the mitochondria-containing isolated extracellular vesicle composition, the value of fasting blood glucose and/or insulin concentration of a rat with diabetes can be reduced, and the insulin resistance can be improved.

Preferably, the medicine is administered to a homeothermic animal or human. Preferably, the homeothermic animal is a mammal or a bird, and the mammal may be a rat or a mouse.

The present invention further provides a use of an isolated extracellular vesicle composition, for use in preparing a medicine for treating or relieving kidney injury. The medicine comprises an effective dose of the mitochondria-containing isolated extracellular vesicle composition and a pharmaceutically acceptable excipient, wherein based on the total amount of the extracellular vesicle, the isolated extracellular vesicle composition comprises 0.2% or more of the mitochondria-containing microvesicle.

The effective dose of the present invention refers to an amount that is effective in achieving the desired treatment or alleviation of chronic kidney disease in terms of dosage and for the required time period. According to the present invention, it means that by administering a specific range of amount of the mitochondria-containing isolated extracellular vesicle composition, the blood creatinine and the urea nitrogen level of a mouse with chronic kidney disease can be reduced.

Preferably, the kidney injury may be chronic kidney disease or acute kidney injury.

Preferably, the medicine is administered to a homeothermic animal or human. Preferably, the homeothermic animal is a mammal or a bird, and the mammal may be a rat or a mouse.

The "pharmaceutical composition or medicine" of the present invention may exist in various forms, the forms include but are not limited to dosage forms of liquid, semi-solid, and solid, such as a solution, an emulsion, a suspension, and any other dosage form similar to or suitable for the present invention.

In one embodiment, the pharmaceutical composition of the present invention is in a form of injection, and the injection is intravenous injection.

The advantages of the present invention include simple, large-scale, continuous production of mitochondrial preparations. Furthermore, the mitochondria-containing microvesicle can be directly isolated from the culture medium, eliminating the need for breaking cells to extract mitochondria or the costly and time-consuming high-speed centrifugation of mitochondria, thus saving both cost and time. Moreover, the collected mitochondria are already encapsulated by the microvesicle and protected by small molecule ribonucleic acid of exocytic factors, making them easier to be stored in cryopreservation.

The isolated extracellular vesicle composition of the present invention can indeed reduce the value of fasting blood glucose and/or insulin concentration of the rat with diabetes, improving insulin resistance, thereby effectively treating or relieving diabetes. Moreover, it can reduce the blood creatinine and the urea nitrogen level of the mouse with chronic kidney disease, thereby effectively treating or relieving kidney injury.

### Description of the Drawings

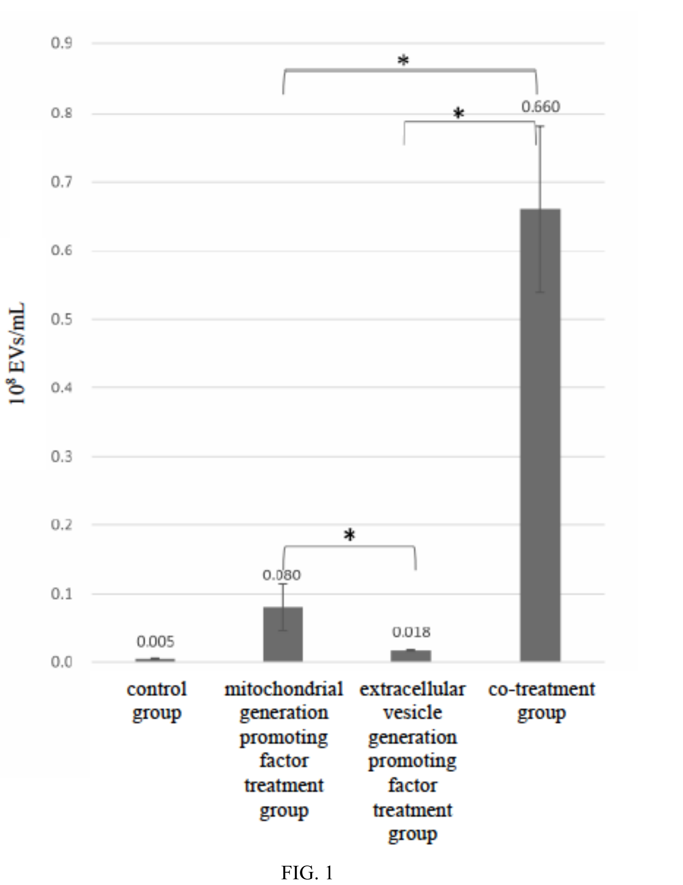

FIG. 1 shows mitochondria numbers in the mitochondria-containing extracellular vesicles produced by human mesenchymal stem cells treated with the mitochondrial generation promoting factor, the extracellular vesicle generation promoting factor, or a combination thereof, where * indicates a confidence interval >95%.

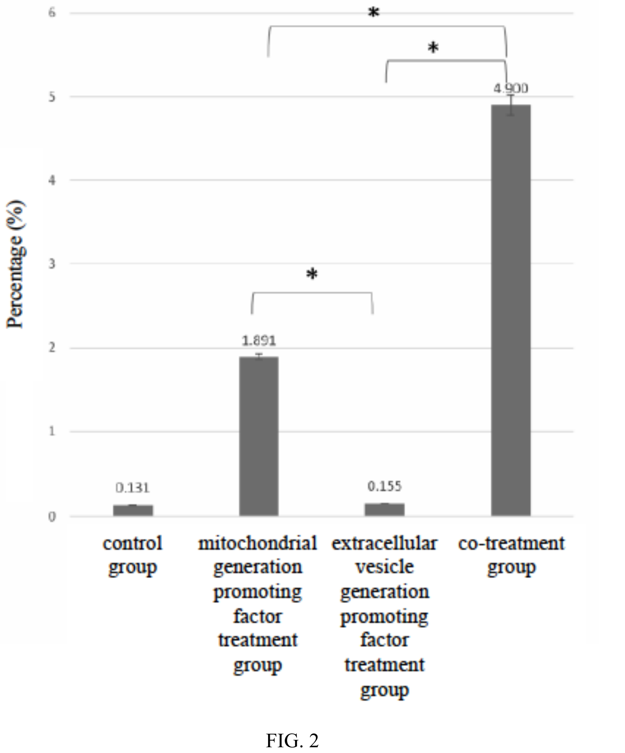

FIG. 2 shows percentages of the mitochondria-containing microvesicle produced by human mesenchymal stem cells treated with the mitochondrial generation promoting factor, the extracellular vesicle generation promoting factor, or a combination thereof, relative to the extracellular vesicles produced in each treatment group, where * indicates a confidence interval >95%.

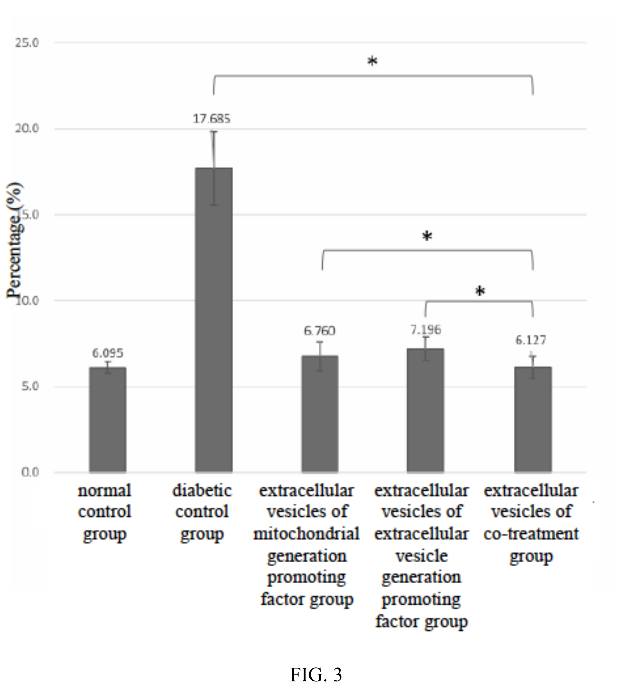

FIG. 3 shows fasting blood glucose value reduction effects in rats with type 2 diabetes of the extracellular vesicles of mitochondrial generation promoting factor, the extracellular vesicles of extracellular vesicle generation promoting factor group, and the extracellular vesicles of co-treatment, where * indicates a confidence interval >95%.

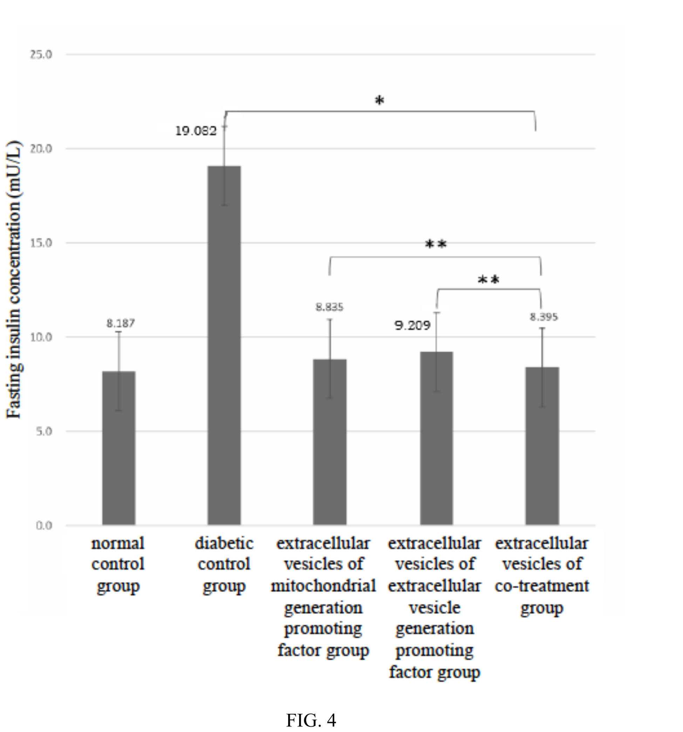

FIG. 4 shows fasting insulin concentration reduction effects in rats with type 2 diabetes of the extracellular vesicles of mitochondrial generation promoting factor, the extracellular vesicles of extracellular vesicle generation promoting factor group, and the extracellular vesicles of co-treatment, where * indicates a confidence interval ≥95% and ** indicates a confidence interval >90%.

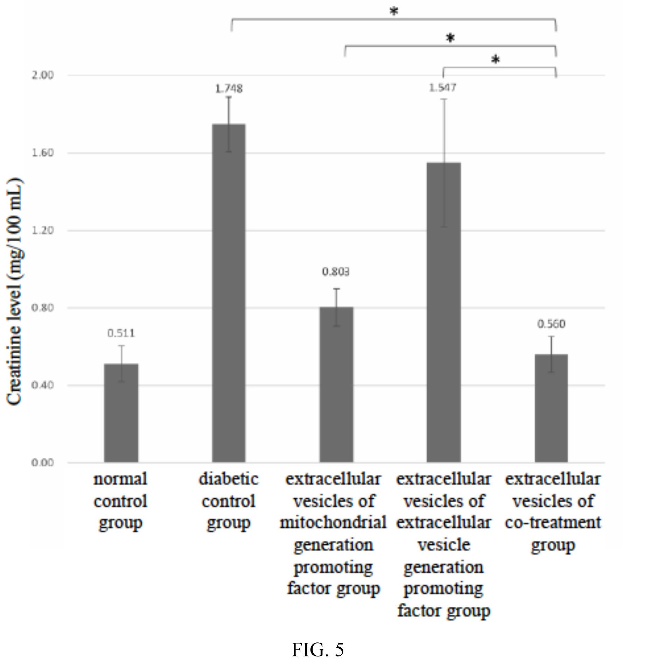

FIG. 5 shows blood creatinine level reduction effects in mice with chronic kidney disease of the extracellular vesicles of mitochondrial generation promoting factor, the extracellular vesicles of extracellular vesicle generation promoting factor group, and the extracellular vesicles of co-treatment, where * indicates a confidence interval >95%.

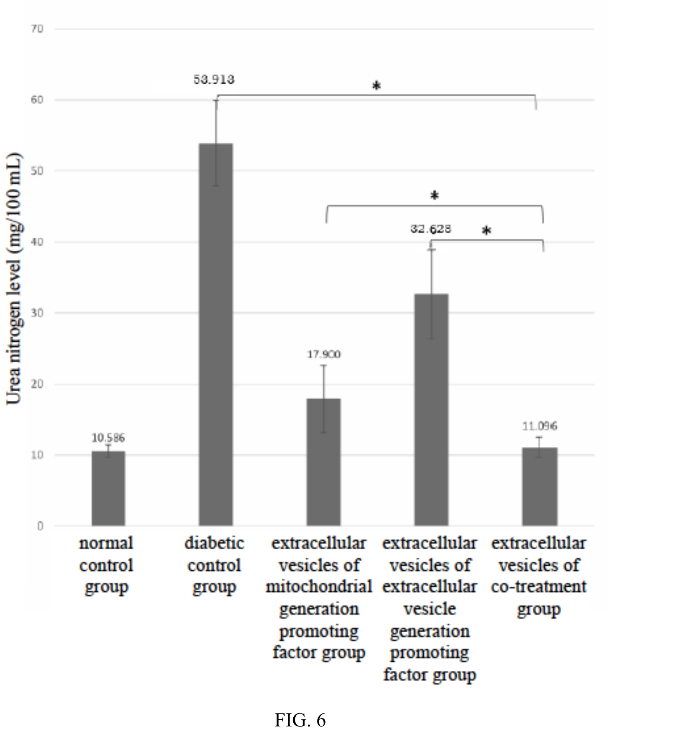

FIG. 6 shows blood urea nitrogen level reduction effects in mice with chronic kidney disease of the extracellular vesicles of mitochondrial generation promoting factor, the extracellular vesicles of extracellular vesicle generation promoting factor group, and the extracellular vesicles of co-treatment, where * indicates a confidence interval >95%.

### Detailed Implementation

The present invention will be further illustrated with the following examples, which do not limit the content previously disclosed in the present invention. Those skilled in the art may make slight improvements and modifications without departing from the scope of the present invention.

### Example 1: Culturing Mesenchymal Stem Cells to Generate Extracellular Vesicles (EVs)

Human mesenchymal stem cells isolated from the umbilical cord of a healthy donor were cultured in a petri dish of 150 millimeters (mm) using high-glucose (glucose concentration of 4500 milligrams (mg)/liter (L)) Dulbecco's Modified Eagle Medium (DMEM) containing 10% fetal bovine serum (FBS) at 5% carbon dioxide and 37°C for 24 hours to 48 hours until the cells reached 40% to 60% confluency. After the cells were washed twice with the aforementioned DMEM medium, the aforementioned DMEM with 10% FBS was added to the cells, and the cells were respectively treated in four groups: a control group, in which the cells were cultured only with the culture medium and received no additional treatment; a mitochondrial generation promoting factor treatment group, in which the mitochondrial generation promoting factor was added to make the concentration of the mitochondrial generation promoting factor in the culture medium reach 40 ng/mL, and the mitochondrial generation promoting factor used in this embodiment was heme oxygenase-1 (Enzo Life Sciences, Ann Arbor, MI, USA); an extracellular vesicle generation promoting factor treatment group, in which the extracellular vesicle generation promoting factor was added to make the concentration of the extracellular vesicle generation promoting factor in the culture medium reach 50 mM, and the extracellular vesicle generation promoting factor used in this embodiment was ethanol; or an extracellular vesicle generation promoting factor and mitochondrial generation promoting factor co-treatment group (short for co-treatment group hereafter), in which the mitochondrial generation promoting factor was added first, and then the extracellular vesicle generation promoting factor was added in this embodiment. Specifically, after the human mesenchymal stem cells were cultured for 24 hours, the mitochondrial generation promoting factor, heme oxygenase-1, was added to make the concentration of heme oxygenase-1 in the culture medium reach 40 ng/mL, and the extracellular vesicle generation promoting factor, ethanol, was added at the 36^{th} hour of cell culturing to make the concentration of ethanol in the culture medium reach 50 mM. After the cells were cultured for 72 hours, supernatants were collected to get the extracellular vesicles of conditioned mediums (CM) generated from each treatment group. Specifically, the conditioned mediums generated from each treatment group were centrifuged at 3,000 g for 10 minutes to remove dead cells or larger cell fragments. Next, the supernatants of the conditioned mediums from each group were pre-cleared using an extracellular vesicle isolation pre-clearing column. The pre-cleared supernatants were transferred to an extracellular vesicle isolation column (Capturem^{™}) and centrifuged at 1,000 g for 2 minutes to 4 minutes at room temperature. The extracellular vesicle isolation column was washed for once with an extracellular vesicle isolation wash buffer, and the extracellular vesicles of each group were eluted with an extracellular vesicle isolation elution buffer in a kit.

### Test Example 1: Nanoparticle Tracking Analysis

The extracellular vesicles obtained after treatment from each group in Example 1 were diluted using a phosphate-buffered saline (PBS) to obtain diluents, which would have 20 to 100 particles in a field of view for analysis. Numbers of the mitochondria-containing microvesicles in the diluents were measured using nanoparticle tracking analysis (NTA), and the final counting of the mitochondria-containing microvesicles were conducted using fluorescent NTA (fNTA). The extracellular vesicles isolated in Example 1 were marked with a tetramethylrhodamine ethyl ester (TMRE) fluorescent dye to detect the mitochondria in the extracellular vesicles. Excitation light wavelength and emission light wavelength of the TMRM fluorescent dye were respectively 550 nanometers (nm) and 575 nm. Sizes of the extracellular vesicles and the mitochondria-containing microvesicles were measured by a nanoparticle analyzer (NanoSight LM10-HS system, Malvern, UK), and data was analyzed using a nanoparticle tracking software (version 2.3).

The results analyzing the numbers of the mitochondria in each treatment group are shown in FIG. 1. Compared to the control group, the mitochondria-containing microvesicles were indeed generated more in the mitochondrial generation promoting factor treatment group, the extracellular vesicle generation promoting factor treatment group, and the co-treatment group. Therefore, when the human mesenchymal stem cells were treated with the mitochondrial generation promoting factor or the extracellular vesicle generation promoting factor, or co-treated with the mitochondrial generation promoting factor and the extracellular vesicle generation promoting factor, the human mesenchymal stem cells can indeed be promoted to generate more mitochondria-containing microvesicles. The human mesenchymal stem cells can be promoted to generate more mitochondria-containing microvesicles in the mitochondrial generation promoting factor treatment group and the co-treatment group, which are respectively 16 times and 132 times of the control group.

In addition, the mitochondria numbers shown in FIG. 2 were divided by the corresponding number of the extracellular vesicles generated in each group to obtain a percentage of the mitochondria-containing microvesicles to the extracellular vesicles generated in each treatment group, as shown in FIG. 2. Compared to the control group, a higher percentage of the extracellular vesicles generated in the mitochondrial generation promoting factor treatment group, the extracellular vesicle generation promoting factor treatment group, and the co-treatment group indeed contain mitochondria. Therefore, when the human mesenchymal stem cells were treated with the mitochondrial generation promoting factor or the extracellular vesicle generation promoting factor, or co-treated with the mitochondrial generation promoting factor and the extracellular vesicle generation promoting factor, the human mesenchymal stem cells can indeed be promoted to generate higher percentages of the mitochondria-containing microvesicles. The human mesenchymal stem cells can be promoted to generate higher percentages of the mitochondria-containing microvesicles in the mitochondrial generation promoting factor treatment group and the co-treatment group, and the percentages are respectively 1.89% and 4.9%, which are respectively 14.44 times and 37.40 times of the control group.

Therefore, using a combination of the mitochondrial generation promoting factor and the extracellular vesicle generation promoting factor can indeed promote cell having mitochondria to generate the mitochondria-containing microvesicles, and the mitochondria-containing microvesicles can indeed be mass-produced in the method of the present invention.

### Test Example 2: Animal Experiment of Type 2 Diabetic Rat Model

Male albino rats weighing 150 grams (g) to 200 g were housed, 7 per cage, and kept in an environment with controlled temperature (22°C to 25°C) and a 12-hour light/dark cycle (light from 08:00 to 20:00). Experimental animals had free access to food and water. On day 0 of the experiment, the experimental animals were randomly divided into 2 groups: a normal control group (n=7) fed with a standard experimental diet for two weeks, and a diabetes group fed with a high-fat diet (HFD; 20% protein, 60% fat, and 20% carbohydrate) for two weeks. On day 14, the experimental animals in the diabetes group were induced type 2 diabetes by intraperitoneal injection of a low-dose streptozotocin (STZ) at 45 milligrams per kilogram (mg/kg). Both the low-dose streptozotocin and the high-fat diet are necessary elements for inducing insulin resistance in type 2 diabetes. Therefore, all rats had free access to their corresponding group's food and water until the end of the experiment. On day 21 of the experiment, values of fasting blood glucose (FBG) and insulin of the normal control group and the diabetes group were measured after fasted overnight. The rats of type 2 diabetic model were further divided into: a diabetic control group (control group), an extracellular vesicles of mitochondrial generation promoting factor group, an extracellular vesicles of extracellular vesicle generation promoting factor group, and an extracellular vesicles of co-treatment group (n=7 per group). Rats of each group received tail vein injections of PBS buffer solution (diabetic control group) twice a week, for a total of two weeks; or tail vein injections of the extracellular vesicles from each treatment group obtained in Example 1, administered twice a week, with each dose being 3×10⁸ extracellular vesicles per kilogram of body weight, for a total of two weeks. During the experiment, all rats were fed with a general diet, and blood samples were collected three weeks after drug administration.

FIG. 3 and FIG. 4 respectively show the improvement in fasting blood glucose and insulin resistance in diabetic rats after each treatment. FIG. 3 shows that in all treatment groups: values of fasting blood glucose of diabetic rats in the extracellular vesicles of mitochondrial generation promoting factor group, the extracellular vesicles of extracellular vesicle generation promoting factor group, and the extracellular vesicles of co-treatment group, were lower than values of fasting blood glucose of rats in the diabetic control group. The effects of the extracellular vesicles of mitochondrial generation promoting factor group and the extracellular vesicles of co-treatment group were better. The extracellular vesicles of mitochondrial generation promoting factor group showed a reduction of 61.78% in the value of fasting blood glucose compared to the diabetic control group, and the extracellular vesicles of co-treatment group showed a reduction of 65.35% compared to the diabetic control group, which was similar to rats in the normal control group.

FIG. 4 shows that in all treatment groups: fasting insulin concentrations of diabetic rats in the extracellular vesicles of mitochondrial generation promoting factor group, the extracellular vesicles of extracellular vesicle generation promoting factor group, and the extracellular vesicles of co-treatment group, were lower than fasting insulin concentrations of rats in the diabetic control group. The effects of the extracellular vesicles of mitochondrial generation promoting factor group and the extracellular vesicles of co-treatment group were better. The extracellular vesicles of mitochondrial generation promoting factor group showed a reduction of 53.70% in the fasting insulin concentration compared to the diabetic control group, and the extracellular vesicles of the co-treatment group showed a reduction of 56% compared to the diabetic control group, which was similar to rats in the normal control group.

Therefore, the mitochondria-containing microvesicles and the extracellular vesicle compositions prepared by the method of the present invention can indeed be used to treat type 2 diabetes, and can reduce the value of fasting blood glucose and improve the high insulin concentration caused by insulin resistance in diabetic rats.

### Test Example 3: Animal Experiment of Chronic Kidney Disease Mouse Model

All animal experiments were conducted in accordance with the Guide for the Care and Use of Laboratory Animals. Six-week-old BALB/c mice were kept in an environment with controlled temperature (25°C) and a 12-hour light/dark cycle. The mice were divided into the following five groups (n=5 per group): (1) a normal control group, (2) a chronic kidney disease control group (treated with PBS), (3) an extracellular vesicles of mitochondrial generation promoting factor group, (4) an extracellular vesicles of extracellular vesicle generation promoting factor group, and (5) an extracellular vesicles of co-treatment group. Herein, the chronic kidney disease in mice was induced by feeding a diet containing 0.25% adenine. After one week, each group of mice received tail vein injections of PBS buffer solution (chronic kidney disease control group) twice a week; or tail vein injections of the extracellular vesicles from each treatment group obtained in Example 1, administered twice a week, with each dose being 3 ×10⁸ extracellular vesicles per kilogram of body weight, for a total of two weeks. During the experiment, all mice were fed with a general diet, and blood samples were collected three weeks after drug administration.

The collected mouse blood was centrifuged at 1500 revolutions per minute (rpm) for 30 minutes, and upper layer serum was taken to measure values of blood creatinine and blood urea nitrogen (BUN) using an enzyme-linked immunosorbent assay (ELISA). The absorbance at 450 nm was measured using a microplate analyzer (Thermo Fisher Scientific).

FIG. 5 and FIG. 6 respectively show the improvement in values of blood creatinine and blood urea nitrogen in chronic kidney disease mice after each treatment. FIG. 5 shows that in all treatment groups: values of blood creatinine of chronic kidney disease mice in the extracellular vesicles of mitochondrial generation promoting factor group, the extracellular vesicles of extracellular vesicle generation promoting factor group, and the extracellular vesicles of co-treatment group, were lower than values of blood creatinine of mice in the chronic kidney disease control group. The effects of the extracellular vesicles of mitochondrial generation promoting factor group and the extracellular vesicles of co-treatment group were better. The extracellular vesicles of mitochondrial generation promoting factor group showed a reduction of approximately 54.06% in the value of creatinine compared to the chronic kidney disease control group, and the extracellular vesicles of co-treatment group showed a reduction of 67.96% compared to the chronic kidney disease control group, which was similar to mice in the cormal control group.

FIG. 6 shows that in all treatment groups: values of blood urea nitrogen of chronic kidney disease mice in the extracellular vesicles of mitochondrial generation promoting factor group, the extracellular vesicles of extracellular vesicle generation promoting factor group, and the extracellular vesicles of co-treatment group, were lower than values of blood urea nitrogen of mice in the chronic kidney disease control group. The effects of the extracellular vesicles of mitochondrial generation promoting factor group and the extracellular vesicles of co-treatment group were better. The extracellular vesicles of mitochondrial generation promoting factor group showed a reduction of approximately 66.80% in the value of blood urea nitrogen compared to the chronic kidney disease control group, and the extracellular vesicles of co-treatment group showed a reduction of 79.42% compared to the chronic kidney disease control group, which was similar to mice in the normal control group.

Therefore, the mitochondria-containing microvesicle prepared by the method of the present invention can indeed be used to treat and improve diabetes, and can reduce kidney injury and the values of blood creatinine and blood urea nitrogen in mice with chronic kidney disease.

In summary, the mitochondria-containing microvesicle can indeed be mass-produced in the method of the present invention. Furthermore, because the isolated extracellular vesicle composition obtained by the method of the present invention comprises a large amount of the mitochondria-containing microvesicle, it can indeed be used to treat or alleviate diabetes, such as type 2 diabetes; and kidney injury, such as chronic kidney disease.

The above is merely for the convenience of explaining a preferred embodiment of the present invention and is not intended to limit the scope of the present invention. The claimed scope of the present invention shall be mainly based on the claims in the patent application.

## Claims

1. A use of a combination of a mitochondrial generation promoting factor and an extracellular vesicle generation promoting factor, for use in promoting a cell having mitochondria to generate a mitochondria-containing microvesicle.

2. The use of the combination of the mitochondrial generation promoting factor and the extracellular vesicle generation promoting factor as claimed in claim 1, wherein the mitochondrial generation promoting factor may be selected from the group consisting of heme oxygenase-1, CoPPIX, hemin, hemin derivatives, mitochondrial structure fragments, cell tissue fragments, and a culture environment having 5% to 20% oxygen concentration.

3. The use of the combination of the mitochondrial generation promoting factor and the extracellular vesicle generation promoting factor as claimed in claim 1, wherein the extracellular vesicle generation promoting factor may be selected from the group consisting of ethanol, EP4 antagonist, endosomal sorting complex required for transport, ARRDC1, associated protein's tumor suppressor gene 101, and cytochalasin B.

4. The use of the combination of the mitochondrial generation promoting factor and the extracellular vesicle generation promoting factor as claimed in claim 1, wherein the cell having mitochondria is a mesenchymal stem cell, a hematopoietic stem cell, a bone marrow stem cell, or a liver cell.

5. A method for generating an extracellular vesicle composition rich in a mitochondria-containing microvesicle, comprising:
Step (1) providing a cell having mitochondria; and
Step (2) co-culturing the cell having mitochondria in a culture medium added with a mitochondrial generation promoting factor and an extracellular vesicle generation promoting factor to obtain the extracellular vesicle composition rich in the mitochondria-containing microvesicle, wherein based on a total amount of the extracellular vesicle, the extracellular vesicle composition rich in the mitochondria-containing microvesicle comprises 0.2% or more of the mitochondria-containing microvesicle.

6. The method for generating the extracellular vesicle composition rich in the mitochondria-containing microvesicle as claimed in claim 5, wherein an addition amount of the mitochondrial generation promoting factor makes a concentration of 0.1 nanograms/milliliter (ng/mL) to 100,000 ng/mL of the mitochondrial generation promoting factor in the culture medium.

7. The method for generating the extracellular vesicle composition rich in the mitochondria-containing microvesicle as claimed in claim 5 or claim 6, wherein an addition amount of the extracellular vesicle generation promoting factor makes a concentration of 10⁻⁷ mmol/liter (mM) to 800 mM of the extracellular vesicle generation promoting factor in the culture medium.

8. An isolated extracellular vesicle composition, wherein based on the total amount of the extracellular vesicle, the isolated extracellular vesicle composition comprises 0.2% or more of the mitochondria-containing microvesicle.

9. The isolated extracellular vesicle composition as claimed in claim 8, wherein the isolated extracellular vesicle composition is prepared by the method as claimed in any one of claims 5 to 7 and obtained through isolation.

10. A pharmaceutical composition, comprising the isolated extracellular vesicle composition as claimed in claim 8 or claim 9 and a pharmaceutically acceptable excipient.

11. A use of an isolated extracellular vesicle composition, for use in preparing a medicine for treating or relieving diabetes, wherein the medicine comprises an effective dose of the isolated extracellular vesicle composition and a pharmaceutically acceptable excipient, wherein based on the total amount of the extracellular vesicles, the isolated extracellular vesicle composition comprises 0.2% or more of the mitochondria-containing microvesicle.

12. The use of the isolated extracellular vesicle composition as claimed in claim 11, wherein the diabetes is type 2 diabetes.

13. The use of the isolated extracellular vesicle composition as claimed in claim 11 or claim 12, wherein the medicine is administered to a homeothermic animal or human.

14. A use of an isolated extracellular vesicle composition, for use in preparing a medicine for treating or relieving renal injury, wherein the medicine comprises an effective dose of the isolated extracellular vesicle composition and a pharmaceutically acceptable excipient, wherein based on the total amount of the extracellular vesicles, the isolated extracellular vesicle composition comprises 0.2% or more of the mitochondria-containing microvesicle.

15. The use of the isolated extracellular vesicle composition as claimed in claim 14, wherein the renal injury is chronic kidney disease.

16. The use of the isolated extracellular vesicle composition as claimed in claim 14 or claim 15, wherein the medicine is administered to a homeothermic animal or human.
